(19) Europäisches Patentamt
European Patent Office
Office européen des brevets

(11) Veröffentlichungsnummer : **0 297 310 B1**

(12) # EUROPÄISCHE PATENTSCHRIFT

(45) Veröffentlichungstag der Patentschrift : **28.08.91 Patentblatt 91/35**

(51) Int. Cl.$^5$ : **A61K 7/32**, A61K 7/48

(21) Anmeldenummer : **88108959.3**

(22) Anmeldetag : **04.06.88**

(54) **Desodorierende und antimikrobielle Zusammensetzung zur Verwendung in kosmetischen oder topischen Zubereitungen.**

(30) Priorität : **24.06.87 DE 3720831**
**27.11.87 DE 3740186**

(43) Veröffentlichungstag der Anmeldung : **04.01.89 Patentblatt 89/01**

(45) Bekanntmachung des Hinweises auf die Patenterteilung : **28.08.91 Patentblatt 91/35**

(84) Benannte Vertragsstaaten : **AT BE CH DE ES FR GB GR IT LI NL SE**

(56) Entgegenhaltungen :
**DE-A- 3 315 058**
**US-A- 2 451 149**
**H.P. FIEDLER: "LEXIKON DER HILFSSTOFFE FÜR PHARMAZIE, KOSMETIK UND ANGRENZENDE GEBIETE", Band 9, Auflage 2, 1981, Seite 439, Editio Cantor, Aulendorf, DE; "Glycerinmonolaurat"**

(73) Patentinhaber : **Beiersdorf Aktiengesellschaft**
**Unnastrasse 48**
**W-2000 Hamburg 20 (DE)**

(72) Erfinder : **Hoppe, Udo, Dr.**
**Lottbeker Weg 7**
**W-2000 Hamburg 65 (DE)**
Erfinder : **Eigener, Ulrich, Dr.**
**Hummelsbütteler Kirchenweg 119**
**W-2000 Hamburg 65 (DE)**
Erfinder : **Sauermann Gerhard, Dr.**
**Hambrook**
**W-2351 Wiemersdorf (DE)**
Erfinder : **Engel, Walter**
**Diesterwegstrasse 22**
**W-2080 Pinneberg (DE)**
Erfinder : **Pape, Wolfgang, Dr.**
**Brantschowstrasse 1**
**W-2000 Hamburg 65 (DE)**

**EP 0 297 310 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine desodorierende und antimikrobielle Zusammensetzung zur Verwendung in kosmetischen oder topischen Zubereitungen, insbesondere desodorierenden kosmetischen Mitteln.

Desodorierende kosmetische Mittel werden insbesondere zur Unterdrückung von unangenehmem Körpergeruch eingesetzt, welcher durch die Einwirkung bestimmter Hautbakterien unter dem Einfluß von Wärme und Feuchtigkeit auf den zunächst weitgehend geruchlosen apokrinen Schweiß infolge der Bildung von stark riechenden Zersetzungsprodukten entsteht.

Zur Zeit sind neben Molekülen, die Gerüche absorbieren, zwei Hauptklassen von Produkten zur Bekämpfung schlechter Gerüche aufgrund des Schwitzens bekannt.

Einmal sind Antitranspirationsmittel auf der Grundlage von Produkten bekannt, die die Schweißbildung unterbinden oder stark hemmen, wie Adstringentien auf der Grundlage von Aluminiumsalzen und insbesondere auf der Grundlage von Aluminiumhydroxychlorid. Mit diesen Mitteln kann man die Bildung schlechter Gerüche unterbinden, indem man ihre unmittelbare Ursache, nämlich die Entwicklung von Schweiß durch die Epidermis unterdrückt (vgl. DE-OS 2137926). Im Gegensatz zu diesen Antitranspirantien handelt es sich bei kosmetischen Mitteln mit desodorierender Wirkung um eine Klasse von Mitteln, die zwar nicht oder nur schwach auf das Schweißvolumen einwirken, die jedoch aufgrund ihrer bakteriziden Wirkung die Bakterien, die zur Zersetzung des Schweißes führen, zerstören. Hierzu zählen Mittel mit einem Gehalt an antimikrobiellen Stoffen. Unter den Verbindungen mit derartigen Eigenschaften wurden z.B. Phenolderivate mit und ohne Halogensubstituenten, organische Quecksilberverbindungen, quartäre Ammoniumverbindungen, wie Cequartyl(R) oder bestimmte Ionenaustauscher oder Metallchelate von 1,3-Diketonen sowie desinfizierend wirkende Abkömmlinge von Aminosäuren bekannt.

Ferner wurden Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel eingesetzt. Phenoxyethanol ist vor allem in saurem und neutralem, aber auch im alkalischen Milieu wirksam und völlig ungiftig. Es gibt bereits in niedrigen Konzentrationen ausreichend Schutz. Aufgrund seines neutralen Geschmacks fand es schnell Eingang in die pharmazeutische und kosmetische Industrie. Seine Wirkung richtet sich allerdings hauptsächlich gegen gram-negative Bakterien.

In chemischer Sicht verhält sich Phenoxyethanol

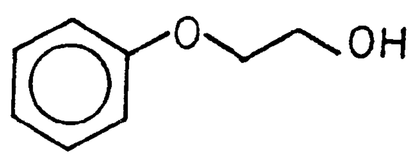

Summenformel : $C_8H_{10}O_2$
Molekulargewicht : 138,17

bei seiner Verwendung weitgehend indifferent. Es stellt eine farblose, leicht viskose Flüssigkeit von schwachem, angenehmen Geruch und einem zusammenziehenden Geschmack dar, ist mischbar mit Aceton, Ethylalkohol und Glycerin, löslich in Wasser (1 : 45) und Fetten, z.B. Oliven- und Erdnußöl (1 : 50).

Die Löslichkeit von Phenoxyethanol in Wasser ist jedoch gering und reicht für manche Konservierungszwecke nicht aus.

Phenoxyethanol, das in der Literatur hinreichend beschrieben ist, wurde in der Natur nachgewiesen in tropischen Früchten, in Cichorium endivia sowie in grünem Tee (Camellia sinesis). Es hat einen milden, rosenähnlichen Duft und wird für Parfümkompositionen auch als Fixatur eingesetzt.

Aus der GB-PS 1155789 ist es auch bekannt, bestimmte Phenylether als antibakterielle Mittel in Reinigungszusammensetzungen für die Haut einzusetzen. Außerdem finden substituierte Phenylether als antibakterielle Mittel Vewendung (vgl. Offenlegungsschrift 1642057).

In Weiterentwicklung des zuvor aufgezeigten Prinzips wurde daher versucht, zusätzlich die antimikrobiellen Eigenschaften bestimmter Riechstoffe, etherischer Öle oder anderer Parfümbestandteile zu nutzen und diese als antimikrobielle und desodorierende Wirkstoffe in desodorierenden Parfümkompositionen einzusetzen. Als eine derartige antimikrobiell wirksame Substanz, die das Wachstum geruchsbildender Bakterien auf der Haut stark hemmt, ohne die gesamte Bakterienflora der Haut stark zu verändern, beschreiben die DE-OS 2728921 und die DE-OS 3315058 den Naturstoff Farnesol (2-trans, 6-trans-3,7,11-Trimethyldodeca-2,5,10-trien-1-ol) und seine 3 synthetischen Isomere. Nachteilig ist dabei jedoch, daß diese Verbindungen bei Verwendung als desodorierender, antimikrobieller Wirkstoff in wesentlich höheren Konzentrationen eingesetzt

werden müssen als in üblichen Parfümzusammensetzungen, um die erwünschte desodorierende Wirkung zu erzielen.

So sind beispielsweise für eine völlige Wachstumshemmung bei den grampositiven Bakterien Staphylococcus aureus und Staphylococcus epidermidis sowie für eine weitgehende Hemmung gegenüber Corynebacterium spec. eine Konzentration von 0,3 Gew.-% Farnesol, bezogen auf die kosmetische Zusammensetzung erforderlich. In Riechstoffkompositionen und in desodorierend wirkenden Produkten beträgt der Gehalt an Farnesol 0,2 bis 0,5%.

Das Farnesol

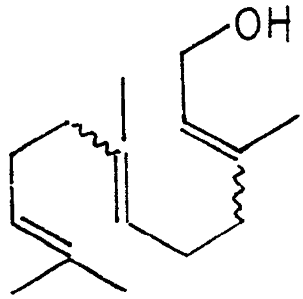

3,7,11-Trimethyldodeca-2,6,10-trienol

Summenformel : $C_{15}H_{26}O$

Molekulargewicht : 222,36

ist ein acyclischer primärer Sesquiterpenalkohol, dessen natürliches Vorkommen in der Literatur hinreichend dokumentiert wurde. So findet man es im Lemongrasöl, Palmarosaöl, Citronellöl, Tuberosenblütenöl, Sandelholzöl, Lindenblütenöl und in vielen anderen Naturstoffen.

Es ist eine farblose Flüssigkeit mit typischem Geruch, klar löslich in 3 Teilen Ethylalkohol (70%).

Als ein für kosmetische Mittel geeignetes Germicid hat auch das Glycerinmonolaurat, bekannt unter der Handelsbezeichnung Lauricidin(R) zu gelten. Es ist dispergierbar in Wasser, löslich in Alkohol, Fetten und Paraffinöl, mischbar mit Aceton.

Glycerin-mono-laurat

$$
\begin{array}{l}
\qquad\quad\; O \\
\qquad\quad\; \| \\
CH_2-O-C-(CH_2)_{10}-CH_3 \\
| \\
CH-OH \\
| \\
CH_2-OH
\end{array}
$$

Summenformel : $C_{15}H_{30}O_4$

Molekulargewicht : 274,41

Glycerinmonolaurat ist in der Natur zumindest als Stoffwechselprodukt bei der Verdauung von Speisefetten nachgewiesen. Verschiedene Monoglyceride sind daher als Zusatzstoffe in der Lebensmittelindustrie üblich. Glycerinmonolaurat selbst findet als pharmazeutische Salbengrundlage, als Co-Emulgator für Emulsionen und als konsistenzgebende Komponente für verschiedenste kosmetische Mittel wie Shampoo, Badezusätze, Cremes oder Lotionen Verwendung.

Diese aufgeführten zwei Klassen von Mitteln sind jedoch nicht vollständig befriedigend, weil einerseits die adstringierenden Mittel oder Antitranspirationsmittel das natürliche Phänomen der Schweißbildung unterbinden und darüber hinaus eine ungünstige Wirkung auf die Epidermis ausüben und andererseits ein Teil der bakteriziden Mittel den Nachteil aufweisen, daß sie vollständig die Mikrobenflora der Haut zerstören und demzufolge das biologische Gleichgewicht der Epidermis empfindlich stören.

Hinzu kommt, daß die Mehrzahl dieser Mittel einen leicht phenolischen Geruch besitzen. Aus diesem Grunde gilt weiterhin das Bestreben, sehr gut desodorierende, geruchsneutrale und von Nebenwirkungen freie kosmetische Mittel herzustellen.

Zwar werden in neuerer Zeit Deodorantien bekannt, welche auf die angeführten traditionellen Wirkstoffe

verzichten. Beispielsweise versucht man das Deo-Problem ausschließlich über das Parfüm zu lösen. Dabei sollen die Körpergeruchskomponenten gewissermaßen als Duftkomplex vom Parfüm so neutralisiert werden, daß der nachteilige Körpergeruch einige Zeit übertönt wird.

Die Wirkung dieser desodorierenden kosmetischen Mittel ist jedoch, was Wirkstärke (Geruchsmaskierung) und Wirkungsdauer angeht, für die Bedürfnisse der Praxis nicht ausreichend.

Weiterhin nutzt man die antibakteriellen Eigenschaften bestimmter Riechstoffe, ätherischer Öle oder anderer Parfümbestandteile einzeln oder in Mischung, indem man desodorierende Parfümkompositionen als solche konfektioniert. Derartige Produkte wirken sowohl über den Duft als auch über die antibakterielle Wirkung über einen längeren Zeitraum desodorierend.

Letztlich ist noch eine Gruppe von Substanzen zu nennen, welche über eine Enzymhemmung verhindern, daß unangenehm riechende Zersetzungsprodukte aus den Schweißinhaltsstoffen, Hornschichtresten und Hautoberflächenfett entstehen.

Aber selbst wenn bei Einsatz von Deodorantien die Gefahr von Hautreizung nicht in dem Maße wie bei Verwendung von Antitranspirantien hervorgerufen wird, so treten auch bei laufender Benutzung von Deodorantien mitunter Unverträglichkeiten, Lichtsensibilisierungen und toxische Nebenwirkungen verschiedener Intensität auf.

Häufiger Nachteil derartiger desodorierender Wirkstoffe ist, daß nicht nur die für den Körpergeruch verantwortlichen Bakterien am Wachstum gehindert oder abgetötet werden, sondern darüber hinaus auch andere Bakterien der bakteriellen Hautflora vernichtet werden. Derartige desodorierende Wirkstoffe sind also in unerwünschter Weise wesentlich stärker wirksam, als zur Vermeidung von Körpergeruch notwendig wäre.

An ein zufriedenstellendes Deo-Mittel werden daher folgende Voraussetzungen geknüpft:

1) Schonung der natürlichen Biologie der Haut
2) Duftneutralität
3) Wirksamkeit nur in Bezug auf Desodorierung, d.h. nur Vermeidung und/oder Beseitigung von Körpergeruch
4) Vermeidung der Bildung von resistenten Bakterienstämmen
5) Vermeidung des sogenannten Stapeleffekts
6) Unschädlichkeit bei Überdosierung oder sonstiger nicht bestimmungsgemäßer Anwendung
7) Gute kosmetische Anwendung und Performance
8) Leichtes Handling (z.B. als Flüssigkeit) und universelle Verwendbarkeit in verschiedensten kosmetischen und externen Zubereitungen.
9) Ausgezeichnete Haut- und Schleimhautverträglichkeit
10) Einsatz umweltfreundlicher Stoffe
11) Rückgriff auf natürliche Systeme oder in der Natur vorkommender Stoffe mit Status (GRAS, RFM etc.)
12) Pufferkapazität

Aufgabe der Erfindung war es daher, eine desodorierende und antimikrobielle Zusammensetzung auf der Basis von in der Natur vorkommenden oder naturnahen Einsatzstoffen, wie z.B. etherischen Ölen oder Duftstoffen, zu schaffen, die bei möglichst weitgehender Schonung der natürlichen Biologie der Haut wirksam desodoriert, universell in verschiedensten desodorierenden kosmetischen Mitteln eingesetzt werden kann und dabei geringere Einsatzmengen erfordert, als der bisher bekannte Stand der Technik vorsieht.

Es wurde gefunden und darin liegt die Lösung dieser Aufgabe, daß eine Zusammensetzung aus einem oder mehreren 3,7,11-Trimethyl-2,6,10-dodecatrien-1-olen, einem Phenylhydroxyalkylether mit maximal 3 C-Atomen im Alkylrest und Glycerinmonolaurat die obengenannten Anforderungen erfüllt.

Gegenstand der Erfindung ist daher eine desodorierende und antimikrobielle Zusammensetzung zur Verwendung in kosmetischen oder topischen Zubereitungen, dadurch gekennzeichnet, daß sie, bezogen auf die Gesamtmenge der Zubereitung

a) 15-45, vorzugsweise 32-36 Gew.-% eines oder mehrerer 3,7,11-Trimethyl-2,6,10-dodecatrien-1-ole,
b) 30-70, vorzugsweise 51-55 Gew.-% eines Phenylhydroxyalkylethers mit maximal 3 C-Atomen im Alkylrest,
c) 5-25, vorzugsweise 9-15 Gew.-% Glycerinmonolaurat

enthält, wobei die Mengen so auszuwählen sind, daß die Summe von a), b) und c) 100% ergibt.

Die verschiedenen 3,7,11-Trimethyl-2,6,10-dodecatrien-1-ole, d.h. der Naturstoff Farnesol und seine geometrischen Isomere, können dabei einzeln oder in Form jeder beliebigen Mischung eingesetzt werden.

Bei den in der erfindungsgemäßen Zusammensetzung eingesetzten Phenylhydroxyalkylether mit maximal

3 C-Atomen im Alkylrest handelt es sich bevorzugt um solche, bei denen sich die Hydroxygruppe am Alkylrest in Position 2 befindet.

Besonders bevorzugt ist der Einsatz von Phenoxyethanol (Ethylenglykolmonophenylether). Die Ethylenglykolmonophenylether werden in Mengen von 30 bis 70 Gew.-%, vorzugsweise 51 bis 55 Gew.-%, einzeln oder in Mischung in der erfindungsgemäßen desodorierenden antimikrobiellen Zusammensetzung verwendet.

Zwar waren auch für Ethylenglykolmonophenylether (U.S. Patent 2,451,149) und Glycerinmonolaurat gewisse antimikrobielle Eigenschaften bekannt, die erfindungsgemäße Zusammensetzung erwies sich aber in überraschender und nicht vorherzusehender Weise als signifikant wirksamer, als dies für die Summe der Einzelkomponenten zu erwarten war.

Bei mikrobiologischen Untersuchungen wurde für mehrere relevante Keime eine synergistische Wirkung der erfindungsgemäßen Zusammensetzung in der Form nachgewiesen, daß die wirksame Menge der erfindunsgemäßen Zusammensetzung geringere Mengen der 3 Komponenten enthielt, als aufgrund der minimalen Hemmkonzentrationen errechnet wurde. Einzelheiten dazu sind in Tabelle 1 wiedergegeben. Die synergistische Wirkung wurde, wie in Beispiel 1 ausführlich beschrieben, anhand des sog. Toxi-Chromotests bestätigt.

Daher ist die erfindungsgemäße desodorierende und antimikrobielle Zusammensetzung bei Verwendung in topischen oder kosmetischen Zubereitungen verglichen mit den Einzelkomponenten auch bei geringeren Einsatzmengen ausreichend wirksam.

Zwar läßt sich ein desodorierender Effekt auch zum Teil für Zusammensetzungen der 3 Komponenten nachweisen, die außerhalb der in Anspruch 1 beanspruchten Zusammensetzungen liegen. Derartige Zusammensetzungen außerhalb der beanspruchten Bereiche erweisen sich in der Praxis aber als ungeeignet, weil die Komponenten dann nicht mehr problemlos mischbar sind und einzelne Komponenten zur Bildung getrennter Phasen neigen, was bei der Einarbeitung in topische oder kosmetische Zubereitungen zu Schwierigkeiten führt. Dies gilt insbesondere bei höheren Anteilen an Glycerinmonolaurat.

Dagegen besteht ein weiterer Vorteil der erfindungsgemäßen Zusammensetzung darin, daß sie problemlos in den verschiedenen Typen von Rezepturen für desodorierende kosmetische Mittel wie Roll -on, Stift, Lotion, Spray oder Lösung eingesetzt werden kann.

Die direkte Einarbeitung der erfindungsgemäßen synergetischen Zusammensetzung in externe Zusammensetzungen und kosmetische Mittel besitzt den Vorteil, daß eine homogene Verteilung der Komponenten gewährleistet ist und somit der zeitaufwendige Einsatz der Einzelkomponenten ausgeschaltet wird.

Eine bevorzugte Ausführungsform der Erfindung stellen daher desodorierende kosmetische Mittel dar, die neben üblichen Bestandteilen als desodorierenden Wirkstoff eine wirksame Menge der erfindungsgemäßen Zusammensetzung enthalten. Als besonders vorteilhaft haben sich bei dieser Ausführungsform der Erfindung desodorierende kosmetische Mittel gezeigt, die vorzugsweise einen Gehalt von 0,05 bis 5,00 Gew.-%, insbesondere 0,10 bis 0,9 Gew.-%, bezogen auf die Gesamtmenge des kosmetischen Mittels der erfindungsgemäßen desodorierenden und antimikrobiellen Zusammensetzung aufweisen.

Chemilumineszenzmessungen auf der Haut legen dabei nahe, daß die gute desodorierende Wirkung der erfindungsgemäßen Zusammensetzung neben der antimikrobiellen Wirkung auch auf oxidative Reaktionen zurückzuführen ist, die durch die erfindungsgemäße Zusammensetzung ausgelöst werden.

Eine weitere vorteilhafte Ausführungsform der Erfindung stellt die Verwendung der erfindungsgemäßen desodorierenden und antimikrobiellen Zusammensetzung als antimikrobieller Wirkstoff zur Stabilisierung topischer oder kosmetischer Zubereitungen gegen die Zersetzung durch Mikroorganismen dar.

Die vorgenommenen mikrobiologischen Untersuchungen (Kontakt-Wachstumsindex nach Heiss) wurden mit den Bakterienarten Staphylococcus aureus ATCC 6538 P, Staphylococcus epidermidis ATCC 12228, E. coli ATCC 8739, Pseudomonas aeruginosa ATCC 9027 und Propionibacterium acnes ATCC 6917 durchgeführt. Dabei wurden jeweils Konzentrationen von 0,1, 0,3 und 1,0% der Verbindungen Glycerinmonolaurat (G) Farnesol (F) und Phenoxyethanol (P) einzeln und in Mischung mit einer Aufschwemmung der Testorganismen ($10^8$-$10^9$ kolonienbildende Einheiten (KBE)/ml) zusammengebracht. Die Durchführung der Untersuchung erfolgte in üblicher Weise, indem Filterpapierplättchen mit einer Fläche von 23,8 $cm^2$ mit je 0,4 g jeweils einer 0,1, 0,3 und 1,0%igen Lösung der Verbindungen (F), (G) und (P) sowie deren Mischungen beaufschlagt wurden. Nach Trocknung der aufgebrachten Lösungen wurden die Filterpapierplättchen in den Nähragar in Petri-Schalen eingebettet, wobei die Oberfläche mit einer dünnen Nähr-Agar-Schicht überschichtet wurde. Anschließend wurde die Platte mit den Testbakterien beimpft (vgl. Tabelle 1).

Die Bewertung der wachstumshemmenden Wirkung durch die Testsubstanzen bzw. deren Mischungen erfolgte an hand der Ziffern 0 bis 4, wo bei Index 4 keinerlei Wirkung aufgezeigt und Ziffer 0 kein Wachstum (totale Hemmwirkung) nachweisbar ist. Der Buchstabe D soll verdeutlichen, daß neben dem fehlenden Wachstum im Bereich der Kontaktfläche des Filterplättchens auch im Randbereich neben dem Filterplättchen nahezu kein Wachstum zu erkennen und somit eine noch bessere Wirkung als beim Index 0 vorhanden ist.

Dabei läßt sich für die Mischungen 1-7 bei Testkonzentrationen von 1,0 und 0,3% eine deutliche antibakterielle Wirkung gegenüber den 3 grampositiven der 5 geprüften Bakterienarten nachweisen, die bei diesen drei Bakterienarten eine totale Wachstumshemmung nach sich gezogen haben.

Auch noch bei einer Einsatzkonzentration von 0,1% der Mischungen 1-7 ist bei den beiden Staphylokokken-Stämmen eine totale bis deutliche Wachstumshemmung erkennbar, während bei dieser Konzentration bei Proionibact. acnes nur eine geringe Hemmung durch die Mischungen 2, 3, 4 und 7 und keine Wachstumshemmung durch die Mischungen 1,5 und 6 nachweisbar ist.

Dies ist überraschend, da das Farnesol allein gegen die beiden Staphylokokken bei Anwendung 0,1% geringer wirksam ist als beispielsweise die Mischungen 3, 5, 6 und 7, in denen es mit nur 15-34% enthalten ist. Das Glycerinmonolaurat, das vergleichbare Wirkung wie die genannten Mischungen aufweist, ist jedoch in diesen Mischungen mit nur 10-25 % enthalten. Phenoxyethanol zeigt weder gegen Staph. aureus noch gegen Staph. epidermidis eine Wirkung.

Überraschend ist auch die ausgeprägte Wirkung der Mischungen gegen Propionibact. acnes, da Farnesol und Phenoxyethanol praktisch keine Wirkung als Einzelsubstanzen gegen diesen Testkeim zeigen und das Glycerinmonolaurat in den Mischungen mit nur 5-25% eingesetzt wird. Von größter Bedeutung ist gerade diese Wirksamkeit, da insbesondere diese Gruppe von Bakterien vorwiegend die Geruchsbildung des Schweißes verursacht.

Tabelle 1 Kontakt-Wachstums-Index (KWI) von Glycerinmonolaurat (G), Farnesol (F) und Phenoxyethanol (P) und deren Mischungen

| Testsubstanz bzw. Mischung | eingesetzte Konzentration % | Anwendung | Kontakt-Wachstums-Index: Staph. aureus | Staph. epidermidis | Propionibact. acnes | Ps. aeruginosa | E. coli |
|---|---|---|---|---|---|---|---|
| Glycerinmonolaurat | 1.0 | 0.4g/ 23,8cm$^2$ | 0 | 0 | 0 | 4 | 4 |
| | 0.3 | | 0 | 0 | 0 | 4 | 4 |
| | 0.1 | | 0 | 0 | 2 | 4 | 4 |
| Farnesol | 1.0 | -"- | 0 | 0 | 3 | 4 | 4 |
| | 0.3 | | 0 | 0 | 4 | 4 | 4 |
| | 0.1 | | 1 | 2 | 4 | 4 | 4 |
| Phenoxyethanol | 1.0 | -"- | 4 | 4 | 4 | 4 | 4 |
| | 0.3 | | 4 | 4 | 4 | 4 | 4 |
| | 0.1 | | 4 | 4 | 4 | 4 | 4 |
| Mischung 1 (G=5% F=45% P=50%) | 1.0 | -"- | 0 | 0 | 0 | 4 | 4 |
| | 0.3 | | 0 | 0 | 0 | 4 | 4 |
| | 0.1 | | 1 | 1 | 4 | 4 | 4 |
| Mischung 2 (G=10% F=35% P=55%) | 1.0 | -"- | 0 | 0 | 0 | 4 | 4 |
| | 0.3 | | 0 | 0 | 0 | 4 | 4 |
| | 0.1 | | 0 | 1 | 3 | 4 | 4 |
| Mischung 3 (G=10% F=30% P=60%) | 1.0 | -"- | 0 | 0 | 0 | 4 | 4 |
| | 0.3 | | 0 | 0 | 0 | 4 | 4 |
| | 0.1 | | 0 | 0 | 3 | 4 | 4 |
| Mischung 4 (G=10% F=20% P=70%) | 1.0 | -"- | 0 | 0 | 0 | 4 | 4 |
| | 0.3 | | 0 | 0 | 0 | 4 | 4 |
| | 0.1 | | 1 | 1 | 3 | 4 | 4 |
| Mischung 5 (G=13% F=34% P=53%) | 1.0 | -"- | 0 | 0 | 0 | 4 | 4 |
| | 0.3 | | 0 | 0 | 0 | 4 | 4 |
| | 0.1 | | 0 | 0 | 4 | 4 | 4 |
| Mischung 6 (G=15% F=15% P=70%) | 1.0 | -"- | 0 | 0 | 0 | 4 | 4 |
| | 0.3 | | 0 | 0 | 0 | 4 | 4 |
| | 0.1 | | 0 | 0 | 4 | 4 | 4 |
| Mischung 7 (G=25% F=25% P=50%) | 1.0 | -"- | 0 | 0 | 0 | 4 | 4 |
| | 0.3 | | 0 | 0 | 0 | 4 | 4 |
| | 0.1 | | 0 | 0 | 2 | 4 | 4 |

Beispiel 1

In einem Lösekessel mit Rühreinrichtung wurde die folgende Mischung zusammengegeben und bei Raumtemperatur gerührt, bis eine homogene Lösung entstanden war :

| | |
|---|---|
| 3,7,11-Trimethyl-2,6,10-dodecatrien-1-ol (Isomerengemisch aus 4 Isomeren, Farnesol Nr. 2/027040 der Fa. Dragoco, Holzminden) | 34 GT |
| Phenoxyethanol | 53 GT |
| Glycerinmonolaurat | 13 GT |

Die antimikrobielle Wirksamkeit der obengenannten erfindungsgemäßen Zusammensetzung wurde im Vergleich zu den Einzelkomponenten mit Hilfe des Toxi-Chromotests (Fa. Orgenics Ltd., Yavne, Israel) untersucht. Die Substanzen wurden als 1%ige Lösungen oder Dispersionen in Wasser in Verdünnungsreihen eingesetzt. Als Kontrolle wurde eine Lösung von Quecksilberchlorid (4 mg/l) verwendet, als interner Standard diente Natriumlaurylsulfat (1 g/l). Es ergaben sich gegenüber den für den Test verwendeten E. Coli-Mutanten die in Tabelle 2 angegebenen minimalen Wachstumshemmkonzentrationen.

Tabelle 2:

| Testsubstanz | minimale Hemmkonzentrationen |
|---|---|
| Quecksilberchlorid | 0,05 ppm |
| Glycerinmonolaurat | 9,8 ppm |
| 3,7,11-Trimethyl-2,6,10-dodecatrien-1-ol (Isomerengemisch alle 4 Isomere) | 156,3 ppm |
| Phenoxyethanol | 10.000,0 ppm |
| Erfindungsgemäße Zusammensetzung | 39,1 ppm |
| Natriumlaurylsulfat | 62,5 ppm |

Tabelle 3 stellt die in der erfindungsgemäßen Zusammensetzung enthaltenen Konzentrationen der Einzelkomponenten zusammen und gibt an, wieviel Prozent der minimalen Hemmkonzentration jeder Einzelkomponente die erfindungsgemäße Zusammensetzung bei der Konzentration enthält, die vollständige Hemmung bewirkt.

Tabelle 3

| Substanz | Konz. in erfindungsgem. Zusammensetzung bei völliger Hemmung | % d. minimalen Hemmkonz. der Einzelsubstanz |
|---|---|---|
| Glycerinmonolaurat | 5,1 ppm | 51,9 % |
| 3,7,11-Trimethyl-2,6,10-docecatrien-1-ol | 13,3 ppm | 8,5 % |
| Phenoxyethanol | 20,7 ppm | 0,2 % |
| | Summe | 60,6 % |

Daraus ergibt sich, daß die wirksame Menge der erfindungsgemäßen Zusammensetzung alle Komponenten in Konzentrationen enthält, die unter der minimalen Hemmkonzentration der jeweiligen Komponente liegen. Addiert man die prozentualen Anteile der Konzentration jeder Komponente an der maximalen Hemmkonzentration, so ergibt sich ein Wert, der deutlich unter den 100% liegt, die bei additivem Verhalten der Komponenten zu erwarten wären.

In der erfindungsgemäßen Zusammensetzung liegt also ein synergistisches Zusammenwirken vor, d.h., die Komponenten verstärken sich gegenseitig in ihrer Wirkung.

Die in den Beispielen 2 bis 9 aufgeführten desodorierenden kosmetischen Mittel wurden nach den für die jeweiligen Mittel üblichen, dem Fachmann bekannten Verfahren hergestellt. Dabei bedeutet die Abkürzung GT Gewichtsteile, die Abkürzung EO steht für Ethylenoxid-Einheiten.

## Beispiel 2:

Deodorant Roll-on:

| | |
|---|---|
| Methylcellulose (Viskontran$^R$ HEC 30 000) | 0,80 GT |
| Wasser | 52,00 GT |
| Ethoxyliertes Glycerinmonococoat 7EO (Cetiol$^R$ HE) | 1,00 GT |
| Hydriertes Rizinusöl 40 EO (Cremophor$^R$ RH40) | 2,50 GT |
| Ethanol | 39,20 GT |
| 1,2-Propylenglykol | 3,00 GT |
| Parfüm | 1,00 GT |
| Erfindungsgem. Zusammensetzung nach Beispiel 1 | 0,15 GT |
| Farblösung 0,025%ig | 0,35 GT |

## Beispiel 3

Desodorierender Stift:

| | |
|---|---|
| 1,2-Propylenglykol | 46,00 GT |
| Stearinsäure | 7,00 GT |
| Ethylalkohol | 35,10 GT |
| Wasser | 10,00 GT |
| NaOH-Plätzchen | 1,20 GT |
| Parfüm | 0,50 GT |
| Erfindungsgem. Zusammensetzung nach Beispiel 1 | 0,20 GT |

## Beispiel 4

Desodorierende Lotion (dickflüssig):

| | |
|---|---|
| Polyethylenglykol(20)oleylether (Cremophor$^R$O) | 2,00 GT |
| Cetylstearylalkohol | 3,00 GT |
| Paraffinöl | 5,00 GT |
| 1,2-Propylenglykol | 3,00 GT |
| Polyvinylpyrrolidon (Luviskol$^R$K30) | 0,50 GT |
| Erfindungsgemäße Zusammensetzung nach Beispiel 1 | 0,15 GT |
| Wasser | 89,90 GT |
| Parfüm | 0,45 GT |

Beispiel 5:

Desodorierende Lotion (dünnflüssig):

| | |
|---|---|
| Ethoxylierter Fettalkohol 6 EO (Cremophor$^R$A) | 1,00 GT |
| Polyethylenglykol(20)oleylether (Cremophor$^R$O) | 1,00 GT |
| Glycerinmonostearat | 2,00 GT |
| Cetylalkohol | 1,00 GT |
| Isopropylmyristat | 2,00 GT |
| Glycerin | 1,00 GT |
| Polyvinylpyrrolidon (Luviskol$^R$K30) | 0,50 GT |
| Erfindungsgem. Zusammensetzung nach Beispiel 1 | 0,15 GT |
| Wasser | 90,90 GT |
| Parfüm | 0,45 GT |

Beispiel 6

Desodorierendes Pumpspray (nicht aerosol)

| | |
|---|---|
| Ethanol | 61,50 GT |
| Ethoxyliertes Glycerinmonococoat 7EO (Cetiol$^R$HE) | 1,50 GT |
| Erfindungsgem. Zusammensetzung nach Beispiel 1 | 0,40 GT |
| Citronensäure | 0,02 GT |
| Wasser | 36,18 GT |

## Beispiel 7

Desodorierendes Körperspray (aerosol)

| | |
|---|---|
| Ethanol | 21,35 GT |
| 1,2-Propylenglykol | 3,00 GT |
| Octyldodecanol (Eutanol$^R$G) | 0,04 GT |
| Parfüm | 0,50 GT |
| Erfindungsgem. Zusammensetzung nach Beispiel 1 | 0,10 GT |
| Isopropylmyristat | 0,01 GT |
| Treibgas | 75,00 GT |

## Beispiel 8

Desodorierende Intim-Waschlösung

| | |
|---|---|
| Cocoamidopropyl-Betain 30%ig (Tego-Betain$^R$L7) | 10,00 GT |
| Ethoxyliertes Glycerinmonolaurat 22EO (Tagat$^R$L2) | 2,00 GT |
| Erfindungsgem. Zusammensetzung nach Beispiel 1 | 0,10 GT |
| Milchsäure 80%ig | 0,50 GT |
| Parfüm | 0,08 GT |
| Wasser | 87,32 GT |

Beispiel 9

Desodorierendes Mittel (flüssig) gegen Haargeruch

| | |
|---|---|
| Polyethylenglykol 400 | 0,20 GT |
| Ethanol | 37,50 GT |
| Parfüm | 0,10 GT |
| Erfindungsgem. Zusammensetzung nach Beispiel 1 | 0,10 GT |
| Hydriertes Rizinusöl 40 EO (Cremophor$^R$RH 40) | 0,20 GT |
| Citronensäure | 0,01 GT |
| Wasser | 61,89 GT |

Beispiel 10

Desodorierende Seife

| | |
|---|---|
| Grundseife 80/20 (ca. 78% Fettsäure) | 96,84 GT |
| Überfettungsmittel | 1,45 GT |
| Farbstoffe | 0,01 GT |
| Antioxydans | 0,05 GT |
| Parfum | 1,07 GT |
| Titandioxid | 0,19 GT |
| Erfindungsgemäße Zusammensetzung nach Beispiel 1 | 0,39 GT |
| | 100,00 GT |

**Patentansprüche**

1. Desodorierende und antibakterielle Zussammensetzung zur Verwendung in kosmetischen oder topischen Zubereitungen, dadurch gekennzeichnet, daß sie, bezogen auf die Gesamtmenge der Zusammensetzung

a) 15-45 Gew.-% eines oder mehrerer 3,7,11-Trimethyl-2,6,10-dodecatrien-1-ole
b) 30-70 Gew.-% eines Phenylhydroxyalkylethers mit maximal 3 C-Atomen im Alkylrest
c) 5-25 Gew.-% Glycerinmonolaurat

enthält.

2. Zusammensetzung nach Anspruch 1, dadurch gekennzeichnet, daß sie, bezogen auf die Gesamtmenge

der Zusammensetzung,

| | |
|---|---|
| a) 32-36 Gew.-% | eines oder mehrer 3,7,11 Trimethyl-2,6,10-dodecatrien-1-ole |
| b) 51-55 Gew.-% | eines Phenylhydroxyalkylethers mit maximal 3 C-Atomen im Alkylrest |
| c) 9-15 Gew.-% | Glycerinmonolaurat |

enthält.

3. Zusammensetzung nach Anspruch 1 oder 2, dadurch gekennzeichnet, daß der Phenylhydroxyalkylether mit maximal 3 C-Atomen im Alkylrest Phenoxyethanol ist.

4. Zusammensetzung nach Anspruch 1, dadurch gekennzeichnet, daß sie, bezogen auf die Gesamtmenge der Zusammensetzung

a) 34 GT eines oder mehrerer 3,7,11-Trimethyl-2,6,10-dodecatrien-1-ole
b) 53 GT Phenoxyethanol
c) 13 GT Glycerinmonolaurat
enthält.

5. Desodorierende kosmetische Mittel, enthaltend neben üblichen Bestandteilen als desodorierenden Wirkstoff eine wirksame Menge der Zusammensetzung nach Anspruch 1.

6. Desodorierende kosmetische Mittel nach Anspruch 5, gekennzeichnet durch einen Gehalt von 0,05 bis 5,0 Gew.-%, bezogen auf die Gesamtmenge des kosmetischen Mittels der Zusammensetzung nach Anspruch 1.

7. Verwendung der Zusammensetzung nach Anspruch 1 als antibakterieller Wirkstoff zur Stabilisierung topischer oder kosmetischer Zubereitungen.

8. Verwendung der Zusammensetzung nach Anspruch 1 als antibakterieller Wirkstoff zur Stabilisierung topischer oder kosmetischer Zubereitungen in einer Menge von 0,5 bis 8,0 Gew.% bezogen auf die Zubereitung.

## Claims

1. Deodorizing and antibacterial composition for use in cosmetic or topical formulations, characterized in that it contains, based on the total amount of the composition,

| | |
|---|---|
| a) 15-45% by weight | of one or more 3,7,11-trimethyl-2,6,10-dodecatrien-1-ols, |
| b) 30-70% by weight | of a phenyl hydroxyalkyl ether with not more than 3 C atoms in the alkyl radical and |
| c) 5-15% by weight | of glycerol monolaurate. |

2. Composition according to Claim 1, characterized in that it contains, based on the total amount of the composition,

| | |
|---|---|
| a) 32-36% by weight | of one or more 3,7,11 trimethyl-2,6,10-dodecatrien-1-ols(sic), |
| b) 51-55% by weight | of a phenyl hydroxyalkyl ether with not more than 3 C atoms in the alkyl radical and |
| c) 9-15% by weight | of glycerol monolaurate. |

3. Composition according to Claim 1 or 2, characterized in that the phenyl hydroxyalkyl ether with not more than 3 C atoms in the alkyl radical is phenoxyethanol.

4. Composition according to Claim 1, characterized in that it contains, based on the total amount of the composition,

a) 34 PW of one or more 3,7,11-trimethyl-2,6,10-dodecatrien-1-ols,
b) 53 PW of phenoxyethanol and
c) 13 PW of glycerol monolaurate.

5. Deodorizing cosmetic agents containing, in addition to customary constituents, an effective amount of the composition according to Claim 1 as the deodorizing active compound.

6. Deodorizing cosmetic agents according to Claim 5, characterized in that they contain 0.05 to 5.0% by weight, based on the total amount of the cosmetic agent, of the composition according to Claim 1.

7. Use of the composition according to Claim 1 as an antibacterial active compound for stabilizing topical

or cosmetic formulations.

8. Use of the composition according to Claim 1 as an antibacterial active compound for stabilizing topical or cosmetic formulations in an amount of 0.5 to 8.0% by weight, based on the formulation.

**Revendications**

1. Composition déodorante et antibactérienne pour utilisation dans des préparations cosmétiques ou topiques, caractérisée en ce qu'elle contient, par rapport à la quantité totale de la composition

| | |
|---|---|
| a) 15-45% | en poids d'un ou de plusieurs triméthyl-3,7,11-dodécatriène-2,6,10-ol-1 |
| b) 30-70% | en poids d'un éther de phénylhydroxyalkyle ayant au maximum 3 atomes de carbone sur le radical alkyle |
| c) 5-25% | en poids de monolaurate de glycérine. |

2. Composition selon la revendication 1, caractérisée en ce qu'elle contient, par rapport à la quantité totale de la composition

| | |
|---|---|
| a) 32-36% | en poids d'un ou de plusieurs triméthyl-3,7,11-dodécatriène-2,6,10-ol-1 |
| b) 51-55% | en poids d'un éther de phénylhydroxyalkyle ayant au maximum 3 atomes de carbone sur le radical alkyle |
| c) 9-15% | en poids de monolaurate de glycérine. |

3. Composition selon la revendication 1 ou 2, caractérisée en ce que l'éther de phénylhydroxyalkyle ayant au maximum 3 atomes de carbone sur le radical alkyle est le phénoxyéthanol.

4. Composition selon la revendication 1, caractérisée en ce qu'elle contient, par rapport à la quantité totale de la composition

| | |
|---|---|
| a) | 34 p. en p. d'un ou de plusieurs triméthyl-3,7,11-dodécatriène-2,6,10-ol-1 |
| b) | 53 p. en p. de phénoxyéthanol |
| c) | 13 p. en p. de monolaurate de glycérine. |

5. Produit cosmétique déodorant contenant, outre les habituels composants, une quantité active de la composition selon la revendication 1, en tant que substance active déodorante.

6. Produit cosmétique déodorant selon la revendication 5, caractérisée par un contenu de 0,05 à 5,0% en poids, par rapport à la quantité totale, du produit cosmétique de la composition selon la revendication 1.

7. Utilisation de la composition selon la revendication 1 en tant que substance active antibactérienne pour la stabilisation de préparations topiques ou cosmétiques.

8. Utilisation de la composition selon la revendication 1 en tant que substance active antibactérienne pour la stabilisation de préparations topiques ou cosmétiques dans une quantité allant de 0,5 à 8,0% en poids par rapport à la préparation.